(19) Europäisches Patentamt
European Patent Office
Office européen des brevets

(11) Publication number: 0 245 719
A2

(12) # EUROPEAN PATENT APPLICATION

(21) Application number: 87106327.7

(22) Date of filing: 01.05.87

(51) Int. Cl.[4]: C07C 51/48 , C07C 51/02 , C07C 53/02

(30) Priority: 15.05.86 IT 2045286

(43) Date of publication of application: 19.11.87 Bulletin 87/47

(84) Designated Contracting States: DE ES SE

(71) Applicant: POLIOLI S.p.A.
Viale Emilia 85
I-20093 Cologno Monzese (Milan)(IT)

(72) Inventor: Bini, Benito
Via Carale di Masera 8
I-28037 Domodossola Novara(IT)
Inventor: Nerci, Elio
Via V. Inama 21
I-20133 Milan(IT)
Inventor: Pirola, Paolo
Via Rocca 50
I-20056 Trezzo Sull'Adda Milan(IT)

(74) Representative: Modiano, Guido et al
MODIANO, JOSIF, PISANTY & STAUB
Modiano & Associati Via Meravigli, 16
I-20123 Milan(IT)

(54) **Method and apparatus for the production of formic acid.**

(57) A method is described for the production of formic acid starting from acqueous solutions of sodium formate. The method comprises the reaction of the formate solutions with sulphuric acid in the presence of water in an amount sufficient to keep the resulting sodium sulphate in solution; the extraction of the solution with an organic solvent selective for formic acid, in particular methyl ethyl ketone and its mixtures with a second solvent; and the rectification of the organic phase with the recovery of formic acid at a concentration of at least 85% by weight. An apparatus for carrying out the above described process is also described.

EP 0 245 719 A2

# METHOD AND APPARATUS FOR THE PRODUCTION OF FORMIC ACID

The present invention relates to a method for the production of formic acid starting from acqueous solutions of sodium formate derived from the production of polyhydric alcohols (neopentylglycol, trimethylolpropane, pentaerythritol, trimethylolethane, etc.). It is also applicable to acqueous solutions of sodium formate derived from processes different from the ones mentioned above, so long as the impurities contained therein are compatible in nature and quantity, with what is provided in the present invention.

As is known, in the production of polyhydric alcohols, each mole of main product also yields one mole of sodium formate (Cannizzaro's reaction). The sodium formate is separated from the main product by using different methods according to the type of polyhydric alcohol prepared. In the case of trimethylolpropane and neopentylglycol, the separation of the sodium formate is achieved by extracting, by means of a solvent, all of the trimethylolpropane or the neopentylglycol together with their organic impurities from the concentrated acqueous solution derived from the reaction, leaving in the acqueous phase all of the sodium formate (Italian patent application N. 24710 A/79).

In the case of pentaerythritol, the sodium formate is found, together with small amounts of pentaerythritol and organic substances, in the bitterns originating from the first crystalization of the pentaerythritol.

The sodium formate thus provided by the above mentioned methods as acqueous solution was mostly used in crystal form to produce formic acid (See, for example, French patent N. 1.429.084 and British patents N. 1.049.013 and N. 1.119.455).

According to said known methods, it is thus first necessary to crystalize the sodium formate starting from solutions thereof, using crystalization methods which are classic but not free from complications.

The crystalline sodium formate is then dispersed in formic acid and transformed, by adding concentrated sulphuric acid, into formic acid and sodium sulphate. This operation is performed continuously in an agitated reactor provided with an exchanger to remove the heat of the reaction. The reaction product, composed of a suspension of sodium sulphate in formic acid, is fed into a thin-layer rotating evaporator.

The formic acid is vaporized in the evaporator and the vapors condense in the appropriate exchanger, while the solid composed of sodium sulphate containing residues of formic acid is continuously discharged from the bottom of the device. The main disadvantages of these methods are:

1) Difficulty in the dosage of the crystalline sodium formate and sulphuric acid, with read possibilities of working with an excess of sulphuric acid or with an excess of sodium formate. In the first case, excess of acid, problems of corrosion arise, especially in the thin-layer evaporator. In the second case, sodium sulphate is obtained which is polluted by sodium formate.

2) Difficulty in providing a constant feed to the thin-layer evaporator, this being due to the nature of the fluid, which is provided in a rather thick suspension.
The inconstancy in the feed turns into an inconstancy in the content of residual formic acid in the sodium sulphate which discharges from the base of the evaporator.

3) Very severe problems of maintenance, mainly caused by the rotating thin-layer evaporators.

4) Presence, in the sodium sulphate obtained, of sodium formate and formic acid in variable amounts.

Similar considerations may also be applied with respect to what is provided in the West German Patent N. 3.137.356, though this last patent does have some improvements with respect to the previous ones.

In the West German patent N. 2.332.137, the obtainment of formic acid is provided, starting from the solution which contains the polyhydric alcohol and the polyoses together with calcium formate. This solution is treated with sulphuric acid to cause the calcium sulphate to precipitate and thus separate, leaving the polyhydric alcohol, the polyoses and the formic acid in solution. This solution is extracted in a countercurrent with a solvent, which is scarcely miscible in water, and which has the property of dissolving formic acid and polyoses, but not polyhydric alcohol. From the organic phase, once the solvent has been recovered, the formic acid is obtained, separated from the polyoses. From the acqueous phase, finally, after removing the solvent contained therein, the solution of polyhydric acohol is obtained, ready for crystalization.

The main limitation of the above mentioned German patent residues in the fact that it is applicable only to the polyhydric alcohol preparation methods which employ calcium hydrate as a catalyst, which, as is known, leads to the formation of calcium formate. Instead, when sodium hydrate is used as a catalyst in the preparation of the polyhydric alcohol, as provided in the present invention, sodium formate is obtained as a by-product and thus, if one attempts to proceed in the manner according to the mentioned patent, sodium sulphate is obtained which ends up, after the extraction process, in the acqueous phase together with the polyhydric alcohol, requiring difficult fractioned crystalization, eliminating, to summarize, the main advantage claimed by the prior patent. Besides, in the example and in the description of the device given in the German patent N. 2.332.137, the possibility is pointed out of obtaining formic acid in concentrations higher than 90% by using, to separate the solvent from the acid, only one rectification column, the solvent in use being extracted from the head thereof, and the formic acid being extracted from the base. Verifications performed by the applicant have proved that this is not possible, since methylethylketone, which is the solvent used, even in the presence of the second solvent cyclohexane, can be separated almost quantitatively only if the formic acid taken from the base of the column has a water content higher than 18-20% (see curves of Fig. 1).

The main aim of the present invention is therefore to provide a method for the production of formic acid starting from acqueous solutions of sodium formate which can be performed continuously and which eliminates the disadvantages of the methods currently in use.

A specific object of the present invention is therefore to provide a process for the production of formic acid which operates in a liquid homogeneous phase, thus avoiding the problems of dosage and manipulation which occur in methods operating in a non-homogeneous phase.

A further object of the present invention is to provide a method for the production of formic acid, which permits obtainment of formic acid in high concentrations of at least 85% and substantially free from impurities different from water.

Not least object of the present invention is to provide a method as described above, which obtains, as a by-product, sodium sulphate sufficiently pure for its use in the field of detergents.

This aim, and these objects, as well as other objects which will become apparent hereinafter, are achieved by a method for the production of formic acid starting from acqueous solutions of sodium formate derived from processes for the preparation of polyhydric alcohols, characterized in that it comprises the phases consisting of:

a) the treatment of said acqueous solutions with sulphuric acid in a quantity substantially equal to the stoichiometric quantity with respect to the formate contained in said solutions, in the presence of an amount of water sufficient to keep the resulting sodium sulphate in solution,

b) the extraction of the solutions deriving from a) in countercurrent with a solvent adapted for selectively dissolving the formic acid formed in phase a), using a ratio, by volume, between said organic solvent and said acqueous solutions ranging from 0.5:1 to 2:1, and separating an organic phase and an acqueous phase, and

c) the rectification of the organic phase deriving from the extraction to obtain formic acid having a concentration of at least 85% and the recovery of said solvent for recycling in the extraction phase b).

According to a further aspect of the present invention, there is provided an apparatus for carrying out the method which is the object thereof.

The method and the apparatus according to the invention will now be described in detail with reference to the accompanying drawings, wherein:

Fig. 1 is a graph, illustrating the distillation curves of mixtures of formic acid/water/MEK which can be obtained in the conventional methods of producing formic acid, as previously described herein, and

Fig. 2 is the block diagram of the apparatus for the production of formic acid according to the present invention.

The method according to the present invention is illustrated as follows:

The solutions of sodium formate deriving from the production of trimethylolpropane and neopentylglycol are used as they are. In fact, since they derive from separation processes by means of extraction in countercurrent with suitable solvents (Italian patent application 24710 A/79) they are essentially composed of sodium formate and water, with contents of other substances in negligible amounts. The solution of sodium formate deriving from the first crystalization of pentaerythritol, instead has the following average composition: sodium formate approximately 40% -pentaerythritol approximately 5% - other organic products approximately 7%. It is thus advantageous, for its proper use in the present method, to eliminate from the solution virtually all of the pentaerythritol and the organic products, by means of a pretreatment of extraction in countercurrent using conventional solvents suitable for dissolving pentaerythritol and organic products, such as tertiary butyl alcohol, tertiary amyl alcohol or secondary butyl alcohol (U.S. patent No. 2.539.737).

The acqueous solutions of sodium formate (1) (see Fig. 2), which can have a formate content which is limited only by the solubility thereof (approximately 45% by weight), e.g., approximately 40%, and with contents of other organic substances preferably below 1% with respect to the formate, are made to react continuously with sulphuric acid (2), in a stoichiometric amount with respect to the formate and at a suitable concentration, in a reactor (a) provided with an agitator and an exchanger, for the removal of the reaction heat, obtaining a solution (3) which on average has the following composition:
Sodium sulphate approximately 20% - Formic acid approximately 14% - Organic products approximately 0.25%.

The starting solutions must in any case contain enough water to keep in solution the $Na_2SO_4$ formed by the reaction and thus achieve one of the advantages of the invention.

The treatment with sulphuric acid does not imply particular technological difficulties since it is a matter of metering perfectly clear solutions of a known composition.

The solution of formic acid (3) thus obtained is fed continuously into the upper part of the extraction column (B) while in the lower part of the same the solvent (4) is fed in countercurrent.

The solvent is composed of methyl ethyl ketone (MEK) or of mixtures thereof with cyclohexane, toluene, benzol or isopropyl formate.

Preferably, a mixture is used of MEK and cyclohexane in mutually variable ratios, ranging in percentual contents of MEK from 99% to 70%, better from 90% to 80%, with water content equal to its saturation.

From the base of the extraction column (B) the acqueous phase of the refined product (6) is drawn, while the organic phase (5), which contains all the formic acid, is continuously taken from the top.

The organic phase (5), in the case in which one starts with the above described percentages of formate and organic residuals, has the following composition:
Formic acid approximately 10%
Water approximately 10%
Other organic substances 0.2%

The acqueous phase of the refined product (6) has a content of: sodium sulphate approximately 27%; formic acid lower than 0.1%; and organic substances lower than 0.05%. The ratio of solvent/acqueous solution of formic acid used in the extraction process ranges between 0.5:1 and 2:1, preferably from 1.5:1 to 1:1.

The theoretical stages of the extraction column must be at least 8. The operating temperatures ranges between 33°C and 55°C, preferably from 40°C to 50°C.

The organic phase (5) is continually fed, together with the recycle of the bottoms (9) of the column (D), in the first rectification column (C) which operates at atmospheric pressure.

From the head of the rectification column (C) the solvent (7) is continually extracted, and recycles at the extraction together with that (12) deriving from the column (E). From the base of the column (C), the diluted formic acid (8) is extracted, approximately 80% of which is still accompanied by organic products (polyoles + polyoses) free from methylethylketone and cyclohexane.

The diluted formic acid (8) is fed continuously in the second rectification column (D) which operates in a vacuum. From the head of the column (D) the formic acid (10) is recovered at a concentration higher than 85%, while from the base formic acid (9) is extracted in a subsequently condensed vapor phase, at a concentration related to the maximum water-formic acid azeotrope at the pressure existing at the base of the column.

The diluted formic acid (9) which exits from the base of the column (D) is recycled in the column (C) as previously described.

From the base of the column (D), finally, a continuous, purge is extracted, this time in the liquid phase (11), to remove the organic substances (polyoles and polyoses).

The acqueous refined product (6) is continually fed into the stripping column (E), from the head of which the solvent (12) contained therein is extracted, which together with that (7) deriving from the column (C) returns to the extraction.

The bottoms (13) of the column (E), composed of an acqueous solution of sodium sulphate at approximately 27%, are sent to the sodium sulphate crystalization apparatus.

The process according to the invention achieves all the intended aims, as is clearly understood from the above description. Indeed, it can be performed continually, is performed in homogeneous phase without the disadvantages related to the presence of solids to be metered or dense suspensions to be manipulated and leads to formic acid with purity greater than 85%, as well as to a valuable by-product, i.e. sodium sulphate with such purity as to be suitable for use in the production of detergents.

Modifications and variations of the inventive process will be apparent to the experts in the field, and are within the scope of the invention claimed herein.

## Claims

1. Method for the production of formic acid starting from acqueous solutions of sodium formate originating from processes for the preparation of polyhydric alcohols, characterized in that it comprises the phases consisting of:

a) the treatment of said aqueous solutions with sulphuric acid in a quantity substantially equal to the stoichiometric quantity with respect to the formate contained in said solutions, in the presence of an amount of water sufficient to keep the resulting sodium sulphate in solution,

b) the extraction of the solutions deriving from a) in countercurrent with a solvent adapted for selectively dissolving the formic acid formed in phase a), using a ratio, by volume, between said organic solvent and said acqueous solutions ranging from 0.5:1 to 2:1, and separating an organic phase and an acqueous phase, and

c) the rectification of the organic phase deriving from the extraction to obtain formic acid having a concentration of at least 85%, and the recovery of said solvent for recycling in the extraction phase b).

2. Method according to claim 1, wherein acqueous solutions of formate containing at the most 1% of organic substances, as residues of the production of polyhydric alcohols, with respect to the sodium formate contained in said acqueous solutions, are subjected to the treatment of phase a) with sulphuric acid.

3. Method according to claim 2, wherein the starting acqueous solutions of formate undergo a pretreatment of extraction with solvent of the residual organic substances to take their content below 1%.

4. Method according to claim 3, wherein the starting acqueous solutions derive from the production of pentaerythritol and said residual organic substances are extracted in the pretreatment phase with solvents selected from tertiary butyl alcohol, tertiary amyl alcohol and secondary butyl alcohol.

5. Method according to any one of the preceding claims, wherein the solvent used in the extraction phase b) is selected from methylethylketone (MEK) and mixtures thereof with cyclohexane, toluene, benzol and isopropyl formate.

6. Method according to claim 5, wherein said solvent comprises a mixture of MEK and cyclohexane with an MEK content of 70-99% by weight.

7. Method according to any one of the preceding claims, wherein, employed in the extraction phase b), is a volume ratio ranging between 1.5:1 and 1:1 between said solvent and acqueous solutions.

8. Method according to any one of the preceding claims, wherein the extraction phase b) is performed at a temperature ranging between 33°C and 55°C.

9. Method according to any one of the preceding claims, wherein the rectification phase c) comprises a first stage of rectification at atmospheric pressure with the separation of solvent from diluted formic acid and organic impurities, and a second stage of vacuum rectification, into which the diluted formic acid is fed, and from which is obtained as final product, formic acid having a concentration of at least 85% by weight and a maximum formic acid-water azeotrope recycled at said first rectification stage.

10. Method according to any one of the preceding claims, comprising a further phase of stripping the acqueous phase separated in the extraction phase b) to separate residues of organic solvent from the acqueous solution containing sodium sulphate.

11. Apparatus for the continuous production of formic acid by means of a process according to claim 1, characterized in that it comprises at least one reactor provided with a mixer for effecting the phase of treatment with sulphuric acid, at least one liquid/liquid extraction column, at least two rectification columns of which a first one operates at atmospheric pressure and a second one operates in vacuum, and recyclings at least of the trailing product of the second rectification column to the head of the first rectification column, and of the solvent exiting from the head of the first rectification column to the extraction column.

12. Apparatus according to claim 11, furthermore comprising a stripping column for the removal of eventual organic solvent from the acqueous phase separated in the extraction phase b).

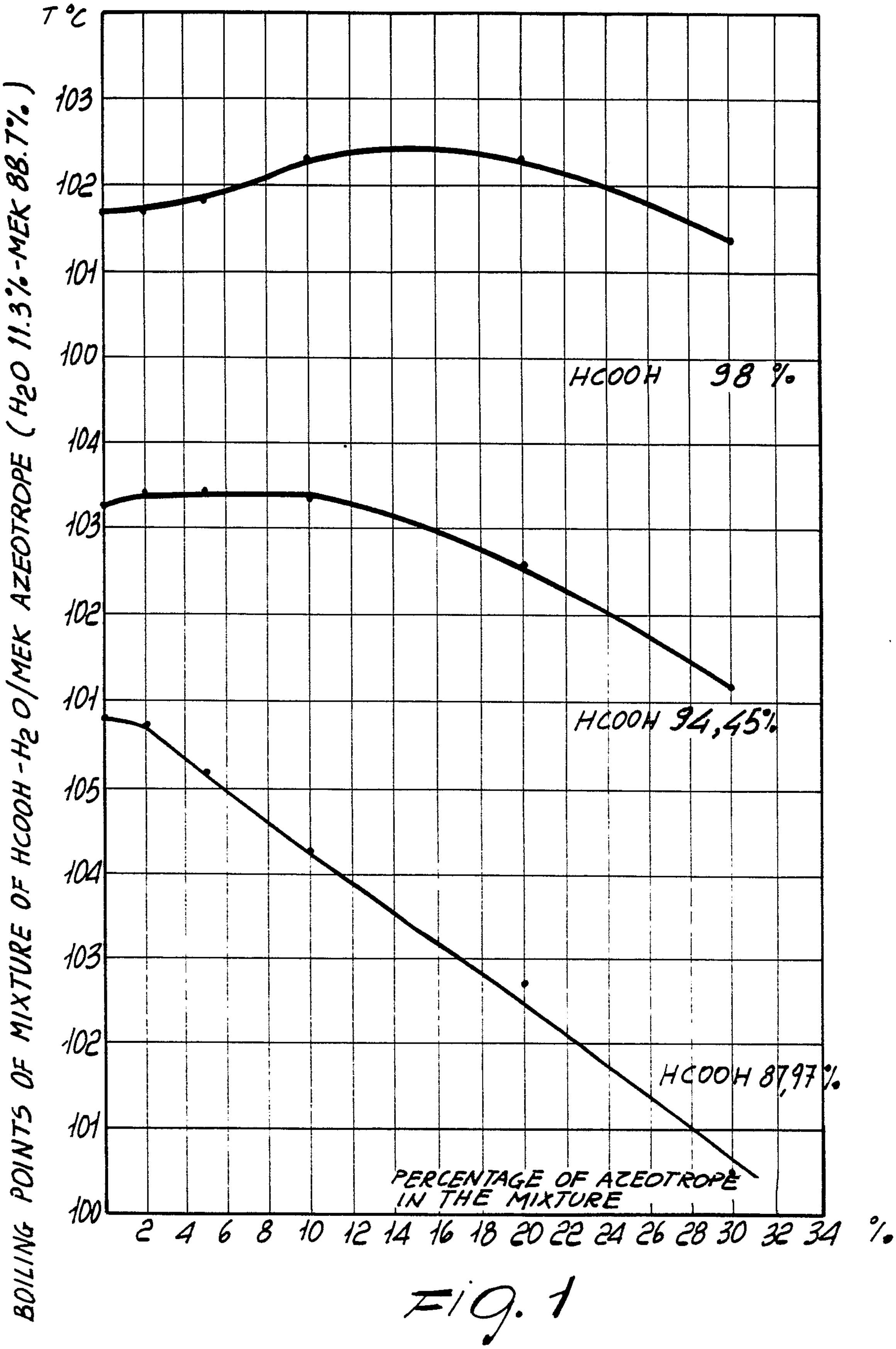
T °C
103
102
101
100
104
103
102
101
105
104
103
102
101
100
HCOOH 98 %
HCOOH 94,45%
HCOOH 87,97%
PERCENTAGE OF AZEOTROPE IN THE MIXTURE
2 4 6 8 10 12 14 16 18 20 22 24 26 28 30 32 34 %
BOILING POINTS OF MIXTURE OF HCOOH-H2O/MEK AZEOTROPE (H2O 11.3%-MEK 88.7%)

Fig. 1

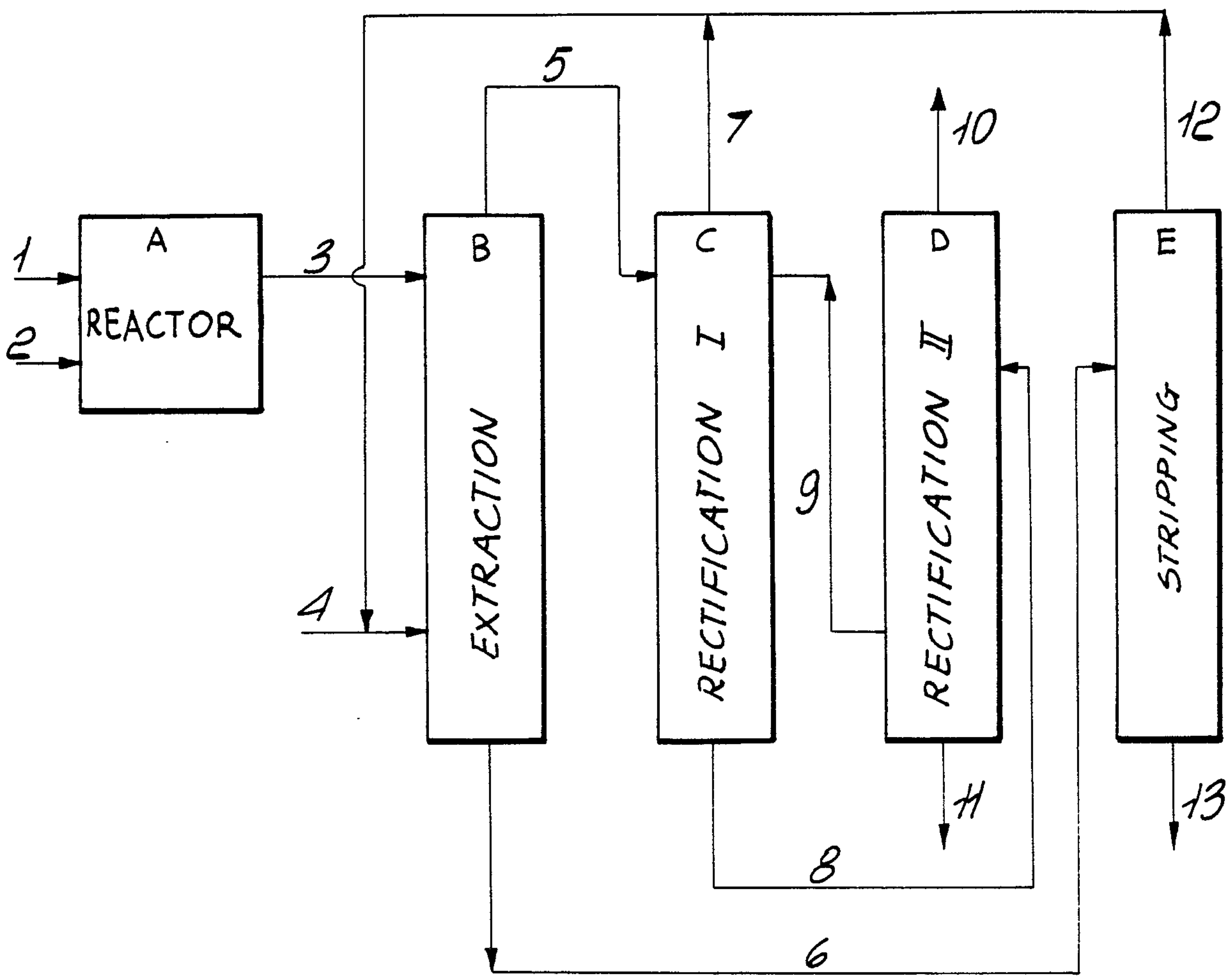
A
REACTOR
B
EXTRACTION
C
RECTIFICATION I
D
RECTIFICATION II
E
STRIPPING
1
2
3
4
5
6
7
8
9
10
11
12
13

Fig. 2